# FASCICULE DE BREVET EUROPEEN

(11) **EP 4 157 424 B1**
(45) Date de publication et mention de la délivrance du brevet: **07.08.2024**
(21) Numéro de dépôt: 21734425.8
(22) Date de dépôt: 01.06.2021
(51) Int. Cl.: A61M 37/00

(54) **APPLICATEUR A MICRO-AIGUILLES**
MIKRONADELAPPLIKATOR
MICRONEEDLE APPLICATOR

(30) Priorité: 02.06.2020 FR 2005784
(43) Date de publication de la demande: 05.04.2023
(73) Titulaire: APTAR France SAS, 27110 Le Neubourg (FR)
(72) Inventeur: DAVIOT, Stéphane, 27110 GRAVERON SEMERVILLE (FR)
(74) Mandataire: CAPRI
(86) Numéro de dépôt international: PCT/FR2021/050988
(87) Numéro de publication internationale: WO 2021/245346

(56) Documents cités:
- WO-A1-2016/049732
- KR-A- 20180 136 722
- US-A1- 2011 295 149
- US-A1- 2012 046 644
- US-A1- 2019 201 615
- US-B1- 6 558 361

## Description

La présente invention concerne un applicateur à micro-aiguilles pour appliquer un produit fluide sur la peau et le faire pénétrer dans la peau. Le domaine d'application de l'invention est celui de la cosmétique, et non pas celui du tatouage. Le but est de faire pénétrer un produit cosmétique dans la peau, et non pas de colorer la peau.

De manière conventionnelle, ce type d'applicateur cosmétique comprend une face d'application pourvue d'au moins une sortie de produit fluide et plusieurs micro-aiguilles. Un moteur électrique est utilisé pour faire vibrer les micro-aiguilles, seules ou avec la face d'application. Un réservoir de produit fluide est relié à la sortie de produit fluide. Le réservoir peut être intégré ou non à l'applicateur. Lorsqu'il est intégré, il se pose alors la question de son remplissage ou de son remplacement. L'applicateur peut être démontable ou comprendre une fenêtre ou un bouchon pour accéder au réservoir.

D'autre part, un organe d'actionnement est aussi prévu pour acheminer le produit fluide du réservoir de produit fluide à la sortie de produit fluide. Cet organe d'actionnement est souvent celui qui commande aussi le moteur, qui cumule alors une double fonction, à savoir de faire vibrer les micro-aiguilles et d'acheminer le produit fluide du réservoir à la face d'application.

US 6,558,361 B1 décrit un applicateur pour transport de fluide avec des micro-aiguilles. L'utilisation de moteur vibrant pour délivrer une substance y est mentionnée.

Le but de la présente invention est de proposer un applicateur à moteur double fonction, dont le réservoir est aisément remplaçable, tout en procurant une liaison simple, efficace et robuste entre le moteur et le réservoir. La manipulation de l'applicateur, et notamment le remplacement du réservoir ne doit pas conduire à une détérioration accidentelle de cette liaison moteur/réservoir.

L'invention est définie dans la revendication indépendante 1. D'autres aspects sont décrits dans les revendications dépendantes 2 à16.

Pour atteindre ce but, la présente invention propose un applicateur à micro-aiguilles pour appliquer un produit fluide sur la peau et le faire pénétrer dans la peau, l'applicateur définissant un axe longitudinal X et comprenant :
- une face d'application pourvue d'au moins une sortie de produit fluide,
- plusieurs micro-aiguilles,
- un réservoir de produit fluide relié à la sortie de produit fluide,
- un moteur pour faire vibrer les micro-aiguilles et acheminer le produit fluide issu du réservoir de produit fluide à la sortie de produit fluide,
caractérisé en ce qu'il comprend deux modules distincts raccordés axialement l'un à l'autre de manière amovible, à savoir :
- un premier module abritant le moteur et des accessoires pour faire fonctionner le moteur, et
- un second module abritant le réservoir de produit fluide, formant la face d'application et supportant les micro-aiguilles.

Ainsi, le second module peut être considéré comme une cartouche ou une recharge que l'on remplace, alors que le premier module avec son moteur est conservé.

Avantageusement, le réservoir est à volume variable, l'actionnement du moteur agissant sur le réservoir sans transmission mécanique de force pour engendrer une diminution de volume du réservoir de produit fluide, de sorte qu'une partie de son contenu est refoulée vers la sortie de produit fluide. En d'autres termes, il n'y a pas de pièce ou d'organe qui relie le moteur au réservoir pour transmettre la force générée par le moteur. Ainsi, il n'y a pas risque d'abimer la transmission, puisqu'elle n'est pas matérielle.

Avantageusement, le premier module peut comprendre une pompe à air. D'autre part, le réservoir de produit fluide peut comprendre une paroi mobile qui fait aussi partie d'une chambre à air alimentée en air pressurisée issu de la pompe à air. De préférence, la chambre à air est formée conjointement par les deux modules raccordés de manière étanche. La transmission peut donc être hydraulique, en opposition à mécanique. C'est air sous pression dans la chambre à air qui va pousser et déplacer la paroi mobile du réservoir, qui peut par exemple se présenter sous la forme d'un piston pousseur ou d'une poche souple. L'astuce est de former cette chambre à air entre les deux modules, de sorte qu'il n'y a pas de pièce ou d'organe de transmission mécanique qui est accessible et donc endommageable, lorsque les deux modules sont séparés l'un de l'autre. Il suffit de créer une étanchéité entre les deux modules pour constituer la chambre à air.

Selon un mode de réalisation pratique, la pompe à air peut comprendre une chambre de pompe dotée d'un clapet d'entrée d'air et d'un clapet de sortie d'air relié à la chambre à air, la variation de volume de la chambre de pompe étant avantageusement assurée par un soufflet. Le soufflet peut être remplacé par un piston coulissant dans un fût. Avantageusement, le clapet d'entrée d'air peut être entrainé axialement en va-et-vient par le moteur. Dans une mise en oeuvre pratique, le moteur peut comprendre un arbre axial entrainé en rotation, un système de transformation de mouvement rotatif en axial étant monté sur l'arbre rotatif. Avantageusement, la pompe à air peut comprendre un piston déplaçable axialement en va-et-vient sous l'action du moteur, ce piston formant un siège pour le clapet d'entrée d'air, un support pour un soufflet et des moyens de transmission pour entrainer les micro-aiguilles du second module en vibration.

Ainsi, un mouvement purement rotatif est transformé en un mouvement vibratoire de va-et-vient pour actionner une pompe à air qui envoie de l'air sous pression dans une chambre à air commune aux deux modules, dont une paroi mobile forme également une partie d'un réservoir à volume variable.

Selon une autre caractéristique de l'invention, le premier module peut comprendre des moyens de neutralisation de la pompe à air pour l'empêcher d'alimenter la chambre à air en air pressurisé, avantageusement en bloquant son clapet d'entrée d'air en position ouverte.

Selon un autre aspect de l'invention, le premier module peut comprendre des moyens d'ajustement de la profondeur de pénétration des micro-aiguilles, agissant avantageusement sur la position axiale du moteur dans le premier module.

Selon encore un autre aspect de l'invention, le premier module peut comprendre des moyens d'embrayage/débrayage pour embrayer/débrayer la transmission des vibrations générées par le moteur aux micro-aiguilles.

Avantageusement, la pompe à air peut être neutralisée lorsque les micro-aiguilles sont embrayées au moteur. De manière symétrique, la pompe à air peut alimenter la chambre à air en air pressurisé lorsque les micro-aiguilles sont débrayées du moteur.

Selon une autre caractéristique, la face d'application est fixe et les micro-aiguilles sont montées sur un porte-aiguilles entrainé en va-et-vient par le moteur.

Avantageusement, les micro-aiguilles sont montées sur un porte-aiguilles qui s'étend autour du réservoir de produit fluide.

L'invention comme définie dans les revendications réside dans le fait de concevoir l'applicateur en deux modules séparables. Cela permet de créer une chambre à air en eux, qui va servir de moyen de transmission de force, sans organe mécanique ou physique, puisque c'est l'air sous pression qui va agir sur le réservoir.

L'invention sera maintenant plus amplement décrite en référence aux dessins joints qui donnent, à titre d'exemples non limitatifs, plusieurs modes de réalisation de l'invention.

Sur les figures :
La figure 1 est une vue en coupe transversale verticale à travers un applicateur de l'invention à l'état assemblé et au repos,
La figure 2 est une vue agrandie tronquée de l'applicateur de la figure 1,
La figure 3 est une vue similaire à celle des figures 1 et 2 à l'état séparé,
Les figures 4a, 4b et 4c sont vues similaires à celle de la figure 1 représentant l'applicateur dans différents états,
La figure 5 est une vue similaire à celle de la figure 3 pour un applicateur selon une variante de réalisation de l'invention, et
Les figures 6a, 6b et 6c sont vues similaires à celles des figures 4a, 4b et 4c pour l'applicateur de la figure 5.

L'applicateur de l'invention est purement cosmétique, voire dermatologique, à l'exclusion du tatouage. Il associe deux moyens de traitement, à savoir la distribution d'un produit cosmétique, qui peut être une crème, une pommade, une lotion, un sérum, etc., et la perforation de l'épiderme, voire du derme, sans toucher l'hypoderme, au moyen de micro-aiguilles. Le produit cosmétique est appliqué avant, en même temps et/ou éventuellement après la perforation. L'applicateur de l'invention est plutôt à usage domestique, en ce sens que l'utilisateur de l'applicateur va l'utiliser sur lui-même. Il peut toutefois s'utiliser de manière professionnelle.

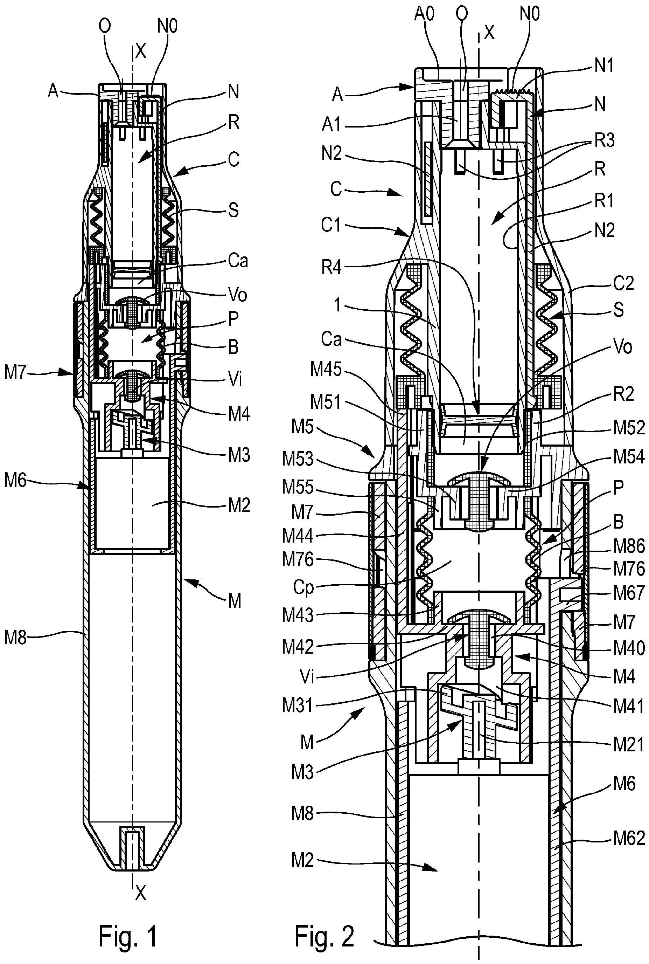

On se référera d'abord aux figures 1 et 2 pour décrire en détail le premier mode de réalisation de l'invention. L'applicateur de l'invention comprend deux modules distincts, à savoir un premier module M et un second module C, qui sont connectables et déconnectables de manière simple et rapide, par l'utilisateur lui-même, par exemple à l'aide de ses deux mains en imprimant un couple et/ou une poussée/traction entre les deux modules. Lorsque les deux modules sont assemblés, l'applicateur présente une configuration générale en forme de stylo avec un axe longitudinal X. L'applicateur peut d'ailleurs être saisi de la même manière qu'un stylo, maintenu entre le pouce et le majeur avec l'index reposant sur l'applicateur.

Le premier module M comprend une coque externe M8 de forme sensiblement cylindrique. La coque M8 est ouverte à son extrémité supérieure et fermée à son extrémité inférieure. La coque M8 comprend une fenêtre latérale M86, qui peut être allongée verticalement pour la réception d'un téton M67, comme on le verra ci-après. La coque externe 8, au niveau de sa fenêtre latérale M86, est entourée par un sélecteur M7, qui se présente sous la forme d'une bague rotative, que l'utilisateur peut faire tourner, comme on le verra ci-après.

Le premier module M renferme un moteur M2, qui est de préférence un moteur électrique. Il peut s'agir d'un petit moteur rotatif qui entraine un arbre M21 en rotation sur lui-même. On peut aussi prévoir un moteur à électroaimant, un moteur linéaire ou un piézoélectrique. Le moteur M2 est alimenté par une batterie (non représentée) et commandé par une électronique (non représentée), qui va gérer la vitesse de rotation de l'arbre M21, les séquences et les durées d'activation du moteur, etc. Un bouton d'activation externe (non représenté) permet à l'utilisateur de mettre l'applicateur sous tension. L'extrémité libre de l'arbre M21 est coiffé d'un capuchon vacillant M3, qui forme une tête inclinée M31. La rotation de l'arbre M21 entraine cette tête inclinée M31 en rotation sur elle-même, décrivant ainsi un mouvement vacillant rotatif.

Avantageusement, le moteur M2 et le capuchon vacillant M3 sont montés sur un chariot mobile M6, qui est mobile selon l'axe longitudinal X. Le chariot mobile M6 comprend un panier M62 qui reçoit le moteur M2 et un téton M67, qui traverse la fenêtre latérale M86 de la coque externe M8 et pénètre dans un chemin de came M76 formé par le sélecteur M7. Le chemin de came M76 est incliné, de sorte que la rotation du sélecteur M7 autour de la coque M8 a pour effet de déplacer axialement le téton M67, du fait qu'il est prisonnier de la fenêtre latérale allongée M86. De ce fait, le chariot M6, avec son moteur M2 et son capuchon M3, est déplacer axialement à l'intérieur de la coque externe M8. La fonction de ce déplacement axial sera décrite ci-après. Le chariot mobile M6 est toutefois optionnel.

Le premier module M comprend également un piston de transmission M4 qui se déplace axialement en va-et-vient. Le piston M4 forme une surface inférieure inclinée M41 qui est en prise avec la tête inclinée M31 du capuchon vacillant M3. Le piston M4 est bloqué en rotation, de sorte que la surface inclinée M41 reste statique en rotation, alors que la tête inclinée M31 est entrainée en rotation par le moteur M2. Il s'ensuit que le piston M4 est entrainé en déplacement axial, du fait du contact vacillant entre la tête inclinée M31 et la surface inclinée M41. Le piston M4 forme un conduit d'entrée M40 et un siège M42 pour un clapet d'entrée d'air Vi. Ce clapet M42 est entouré par un support de soufflet M43. Le piston M4 comprend également des moyens de transmission M44, qui peuvent se présenter sous la forme de plusieurs (entre 2 et 6) pattes ou tiges axiales définissant des extrémités libres de contact M45. Les moyens de transmission M44 peuvent coulisser en contact de la paroi interne de la coques externe M8.

Le premier module M comprend aussi une virole M5 qui définit une partie d'une chambre à air Ca. La virole M5 comprend un cylindre M51 qui peut être revêtu intérieurement par une gaine d'étanchéité M52. La virole M5 forme un conduit de sortie M53 et un siège M54 pour un clapet de sortie d'air Vo. La virole comprend aussi un support de soufflet M55. La virole M5 est maintenue fixement dans la coque externe M8 par une manchette M56. La virole M5 peut servir à bloquer le piston de transmission M4 en rotation. Les extrémités de contact M55 des moyens de transmission M44 du piston M4 peuvent par exemple passer à travers des fenêtres de la virole M5 autour du cylindre M51.

Selon l'invention, le premier module M intègre une pompe à air P, qui est définie entre le piston de transmission M4 et la virole M5. Plus précisément, la pompe à air P comprend un soufflet B qui est monté de manière étanche sur les deux supports de soufflet M43 et M55. Une chambre de pompe Cp est ainsi définie. Elle est alimentée en amont par de l'air passant par le conduit d'entrée d'air M40 et contrôlé par le clapet d'entrée d'air Vi. L'air comprimé lors de l'écrasement du soufflet B est refoulé à travers le conduit de sortie d'air M53 qui est contrôlé par le clapet de sortie Vo. L'écrasement du soufflet B résulte du déplacement axial du piston M4 par rapport à la virole M5, qui reste statique. Le soufflet B est ainsi successivement écrasé et étiré par le déplacement en va-et-vient du piston M4, qui est soumis à la rotation vacillante du capuchon M3 entrainé par le l'arbre M21 du moteur M2. On comprend alors que de l'air sous pression est distribué par la pompe à air P dans la chambre à air Ca.

L'actionnement du sélecteur M7 a pour effet de déplacer axialement le moteur M2, son capuchon M3 ainsi que le piston M4, ce qui fait varier le volume de la chambre de pompe Cp et étend ou rétracte les extrémités de contact M45.

Le second module ou cartouche C ne contient aucun organe ou composant électrique ou électronique : on peut dire qu'il est passif et donc peu coûteux. Ce second module C intègre un réservoir de produit fluide R qui contient un produit fluide, de préférence cosmétique, qui présente avantageusement une haute viscosité : il peut s'agir d'un gel, d'une crème, d'une pommade, d'une huile, etc. Le réservoir est à volume variable : cela signifie que son volume utile diminue à mesure que du produit fluide en est extrait. Le produit fluide reste donc hors de contact de l'air. Le réservoir comprend une paroi mobile sur laquelle on peut exercer une force pour mettre de produit fluide contenu dans le réservoir sous pression et refouler une partie vers l'extérieur. La paroi mobile sert donc de moyens de mise sous pression du réservoir, à la différence d'une paroi mobile qui se déplace en réponse à une dépression générée dans le réservoir. Selon l'invention, la paroi mobile du réservoir fait également partie de la chambre à air Ca, définie précédemment.

Dans le mode de réalisation utilisé pour illustrer l'invention, la paroi mobile est un piston pousseur R4 qui se coulisse de manière étanche dans un fût de coulissement R1, qui définit à son extrémité inférieure une douille d'étanchéité R2, destinée à venir en prise étanche dans la gaine d'étanchéité M52 de la virole M5, de manière à constituer la chambre à air Ca. Avantageusement, le fût R1 comprend plusieurs nervures d'éventation R3 à son extrémité supérieure pour créer un chemin de fuite lorsque le piston pousseur R4 arrivera à leur niveau. On évite ainsi de créer une surpression excessive dans la chambre à air Ca. On peut voir sur les figures que le fût R1 est formé par un corps C1 qui forme également un carénage externe C2 qui enveloppe le fût R1 en définissant entre eux des espaces de réception. Un ressort de rappel S est logé dans l'espace inférieur ; son extrémité supérieur est relié fixement au corps C1, alors que son extrémité inférieure est libre et peut donc se déplacer axialement en comprimant le ressort. L'extrémité inférieure du ressort S est connectée à des moyens de transmission N2, qui peuvent se présenter sous la forme de plusieurs (entre 2 et 6) pattes ou tiges axiales qui peuvent coulisser entre le fût R1 et le carénage C2. Ces moyens de transmission N2 font partie intégrante d'un porte-aiguilles N qui forme également une face de support N1 pourvue de plusieurs micro-aiguilles NO. Leur nombre peut varier de 5 à 100. Leur épaisseur peut varier de 0,05 à 0,5 mm. Leur longueur peut varier de 0,1 à 0,7 mm. Ces valeurs sont données à titre purement indicatif. La face de support N1 est située axialement au-dessus du réservoir R, sur le côté droit des figures. On comprend que les micro-aiguilles NO peuvent se déplacer axialement vers le haut à l'encontre de la force élastique exercée par le ressort de rappel S. En position de repos, la face de support N1 est en butée contre le haut du réservoir.

On peut remarquer que le réservoir R occupe une position centrale ou axiale, alors que les moyens de transmission N2 s'étendent autour et le long du réservoir R.

Le second module ou cartouche C comprend également un embout d'application A, qui est monté fixement au sommet du fût R1, qui forme une cheminée de montage R5 à cet effet. L'embout d'application A forme un canal de sortie A1 qui définit une sortie de produit fluide O qui débouche au niveau d'une face d'application AO. Ainsi, le produit fluide stocké dans le réservoir R est mis sous pression par le déplacement du piston pousseur R4 qui est soumis à la surpression régnant dans la chambre à air Ca. En réponse, une partie du produit fluide est refoulée à travers le canal de sortie A1 et la sortie O pour parvenir sur la face d'application AO.

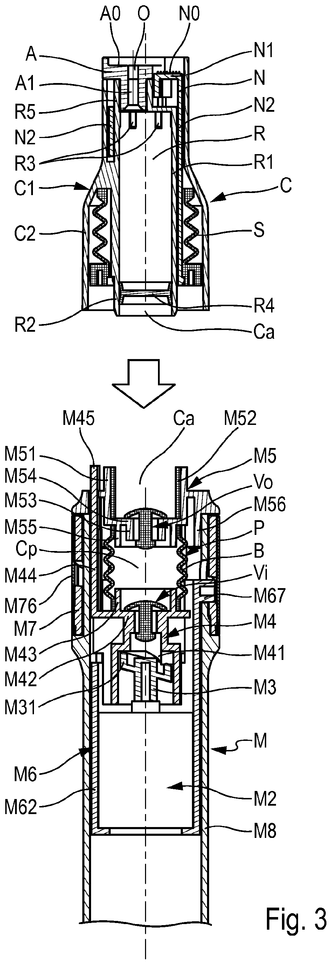

Sur la figure 3, on voit que les deux modules M et C sont deux entités distinctes, qui peuvent être raccordées axialement avec un déplacement relatif indiqué par la flèche visible entre elles. Lors de ce déplacement, la douille d'étanchéité R2 pénètre dans la gaine M52 en créant un contact étanche entre elle : la chambre à air Ca est ainsi constituée. En même temps, le bord inférieur du carénage C2 vient en contact de butée ou d'encliquetage avec la virole M5 : une connexion stable est ainsi établie. L'applicateur est alors dans l'état monté de repos représenté sur les figures 1 et 2, avec le sélecteur M7 en position basse. Le capuchon vacillant M3 est dans une position angulaire qui correspond au point mort bas de la course du piston de transmission M4. La face de support N1, avec ses micro-aiguilles NO, est alors en position basse maximale. On peut remarquer sur les figures 1 et 2 que la face de support N1 est décalée axialement vers le bas par rapport à la face d'application AO. La chambre de pompe Cp est dans état de volume maximal. D'autre part, on peut voir que les extrémités de contact M45 des moyens de transmission M44 du piston M4 sont en contact avec l'extrémité inférieure du ressort de rappel S, qui est reliées aux moyens de transmission N2 du porte-aiguilles N.

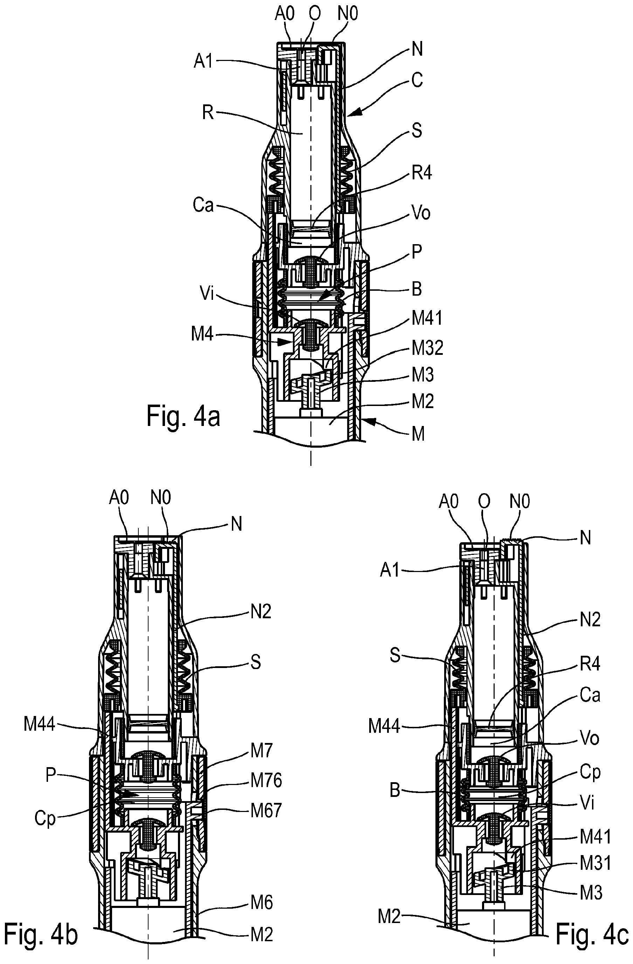

Sur la figure 4a, l'applicateur des figures 1 et 2 a été actionné : son moteur M2 a effectué un demi-tour, ce qui a pour effet d'avoir fait tourner le capuchon vacillant M3 qui a poussé le piston de transmission M4 dans sa position haute maximale. Le déplacement axial du piston M4 a deux effets distincts, mais simultanés. Le premier effet est de transmettre la force exercée par le moteur M2 au travers du capuchon vacillant M3, qui transforme une rotation en déplacement axial, directement au porte-aiguilles N, de sorte que les micro-aiguilles NO se mettent à vibrer axialement. Le second effet est d'actionner la pompe à air P, qui va envoyer de l'air sous pression dans la chambre à air Ca, ce qui va pousser le piston pousseur R4 dans le fût R1 pour refouler du produit fluide sur la face d'application AO. La figure 4a montre l'applicateur lorsque la face de support N1 est dans sa position la plus étendue et la chambre de pompe dans son état de volume minimal.

Sur la figure 4b, le sélecteur M7 a été actionné : le téton M67 du chariot M6 a été entrainé vers le haut sous la contrainte du chemin de came M76. Le moteur M2, son capuchon M3 et le piston de transmission M4 ont été déplacés axialement vers le haut. Le soufflet B a été légèrement écrasé, de sorte que le volume utile maximal de la chambre de pompe Cp a légèrement diminué. Par ailleurs, le piston de transmission M4 a déplacé le porte-aiguilles N, de sorte que la face de support N1 est plus haute que sur les figures 1 et 2 : elle est sensiblement ou exactement au même niveau que la face d'application AO, alors que le piston de transmission M4 est en position basse.

Sur la figure 4c, l'applicateur de la figure 4b a été actionné d'un demi-tour. La chambre de pompe Cp est dans son état de volume minimal et la face de support N1 est en position étendue maximale. On voit qu'elle est plus haute que sur la figure 4a. Le produit fluide est distribué au niveau de la face d'applicateur par mise sous pression de la chambre à air Ca et déplacement du piston pousseur R4, alors que les micro-aiguilles NO vont pénétrer plus profondément dans la peau en raison de leur position avancée ou étendue.

Le sélecteur M7 agit donc sur la profondeur de pénétration des micro-aiguilles NO. L'utilisateur peut lui-même ajuster cette profondeur en fonction de différents paramètres, tels que la nature de la peau, sa souplesse, la nature du produit fluide, le résultat recherché, etc.

On peut surtout noter que la transmission de force entre les deux modules est assurée sans liaison mécanique, telle qu'une tige ou un tube qui ferait saillie hors du premier module pour pouvoir pénétrer dans le second module et pousser la paroi mobile du réservoir. Une telle liaison mécanique pourrait être endommagée lorsque les deux modules sont séparés. De plus, il faudrait repousser ou ramener cette liaison mécanique dans sa position initiale à chaque remplacement de cartouche. Avec l'invention qui prévoit une transmission non mécanique, tous ces inconvénients sont éliminés. L'air sous pression est un moyen de transmission de force qui ne peut pas être endommagé et qui n'a pas besoin d'être ramené en position initiale.

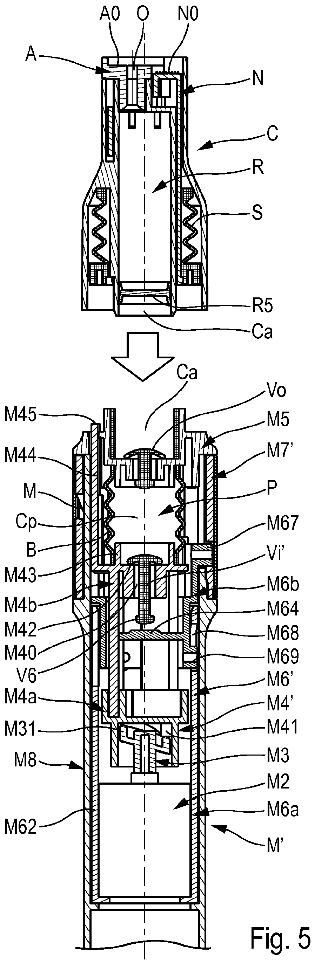

On se référera maintenant à la figure 5 pour décrire une variante de réalisation d'un applicateur selon l'invention. L'applicateur est toujours réalisé en deux modules distincts séparables. Le second module ou cartouche C peut être similaire ou identique à celui du premier mode de réalisation. Et ne sera donc pas décrit. Quant au premier module M', le moteur M2, le capuchon vacillant M3, la virole M5, le clapet de sortie d'air Vo et la coque externe M8 peuvent être similaires ou identiques à ceux du premier mode de réalisation. Même le sélecteur M7' peut être identique, mais avec une fonction différente. Le piston de transmission M4 du premier mode de réalisation a été remplacé par un piston de transmission M4' en deux pièces, à savoir une partie inférieure M4a et une partie supérieure M4b. De même, le chariot M6 du premier mode de réalisation a été remplacé par un chariot M6' en deux pièces, à savoir un panier M6a et un actionneur de clapet M6b.

Plus en détail, la partie inférieure M4a du piston de transmission M4' forme la surface inclinée M41 qui vient en prise avec la tête inclinée du capuchon vacillant M3. La partie supérieure M4b est montée fixement sur la partie inférieure M4a et forme un conduit d'entrée M40, un siège M42 pour un clapet d'entrée d'air Vi', un support de soufflet M43 et des moyens de transmission M44 définissant des extrémités libres de contact M45, tout comme dans le premier mode de réalisation.

Le panier M6a du chariot M6' forme un logement M62 pour le moteur M2, son capuchon vacillant M3, la partie inférieure M4a du piston de transmission M4' et aussi la portion inférieure de la partie supérieure M4b. L'actionneur de clapet M6b est monté à coulissement limité dans le panier M6a. Il comprend un téton M67 en prise dans le chemin de came M76 du sélecteur M7', ainsi qu'un doigt de poussée M64 qui s'étend en-dessous du clapet d'entrée d'air Vi'. A cet effet, le clapet Vi' comprend un talon inférieur V6 qui est destiné à venir en contact avec le doigt de poussée M64 pour décoller le clapet Vi' de son siège M42, comme on le verra ci-après. Le panier M6a forme ouverture allongée M68 et l'actionneur de clapet M6b comprend un picot M69 en prise dans l'ouverture allongée M68, de manière à pouvoir se déplacer axialement sur une course limitée.

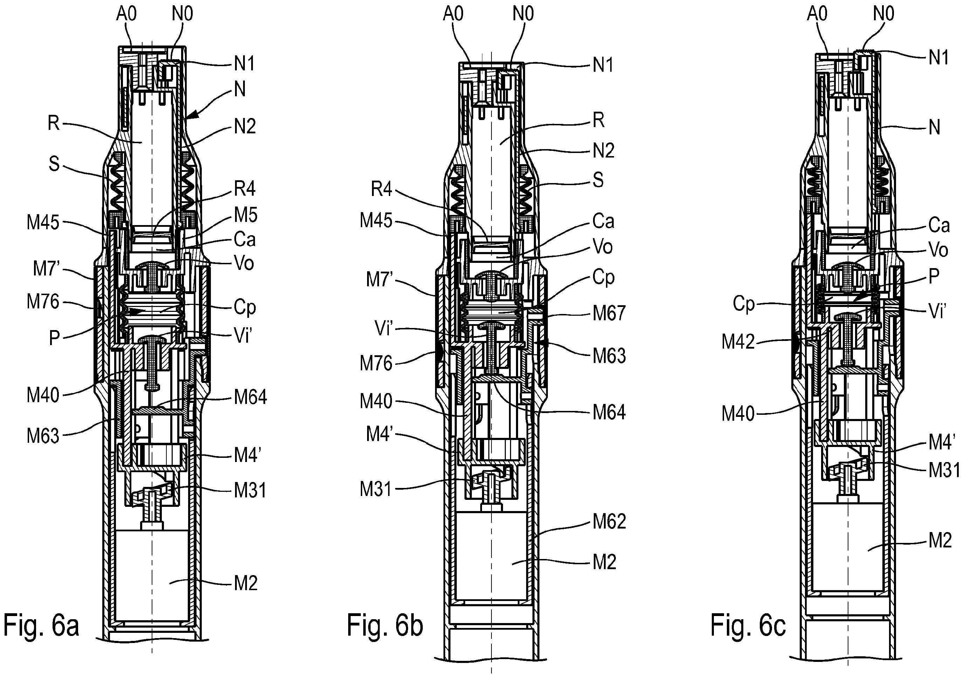

La figure 6a est similaire à la figure 4a : Le sélecteur M7' est en position basse et le moteur M2 a tourné d'un demi-tour, de sorte que le piston M4' est en position haute maximale. La pompe P a été actionnée et de l'air sous pression a été injecté dans la chambre à air Ca pour pousser le piston pousseur R4 et refouler du produit fluide sur la face d'application AO. Cependant, le porte-aiguilles N est reste statique, étant donné que les extrémités de contact M45 sont hors de contact du porte-aiguilles N, et cela même dans la position haute du piston M4'. On peut en effet remarquer sur la figure 6a qu'il persiste un petit intervalle entre l'extrémité M45 et le ressort de rappel S. On peut aussi noter que le ressort est en butée sur la virole M5. Ainsi, l'applicateur est dans une configuration dans laquelle il ne distribue que du produit fluide : la pompe à air fonctionne normalement, alors que les micro-aiguilles NO restent statiques ou inactives. L'utilisateur peut donc se servir de l'applicateur sans micro-perforation.

Sur la figure 6b, le sélecteur M7' a été actionné : le téton M67 du chariot M6' a été entrainé vers le haut sous la contrainte du chemin de came M76. Le moteur M2, son capuchon M3 et le piston de transmission M4 ont été déplacés axialement vers le haut. L'extrémité de contact M45 du piston de transmission M4 est maintenant en contact de l'extrémité inférieure du ressort de rappel S. Le porte-aiguilles N a été déplacé vers le haut, de sorte que la face de support N1 est maintenant sensiblement ou exactement au même niveau que la face d'application AO, alors que le piston de transmission M4' est en position basse par rapport au capuchon vacillant M3. En même temps, le doigt de poussée M64 a décollé le clapet d'entrée d'air Vi' de son siège, de sorte qu'il ne peut plus remplir sa fonction d'étanchéité sélective pendant les phases de surpression dans la chambre de pompe Cp. Le déplacement du doigt de poussée M64 par rapport au piston M4' est possible, car l'actionneur de clapet M6b se déplace sur une petite course axiale limitée par rapport au panier M6a.

Sur la figure 6c, l'applicateur de la figure 6b a été actionné d'un demi-tour. Le capuchon M3 et le piston M4' ont été déplacés vers le haut, comprimant le ressort S et déplaçant le porte-aiguilles N. On voit que la face de support N1 est plus haut que sur la figure 6b et que les micro-aiguilles NO font saillie par rapport à la face d'application AO. Par contre, la pompe à air P n'a pas envoyé d'air sous pression dans la chambre à air Ca. En effet, son clapet d'entrée Vi' est décollé de son siège M42, de sorte que la chambre de pompe Cp ne peut pas être mise sous pression. L'air qui rentre par le clapet d'entrée Vi' en sort à nouveau dès que le soufflet B est écrasé. Ainsi, l'applicateur est dans une configuration dans laquelle il ne distribue pas de produit fluide : seules les micro-aiguilles NO restent sont actives. L'utilisateur peut donc se servir de l'applicateur sans distribution de produit fluide.

Le sélecteur M7' agit tel un moyen de permutation permettant de passer d'une distribution de produit fluide à une micro-perforation, sans jamais pouvoir mettre en oeuvre les deux simultanément.

En variante, une permutation entre une distribution (seule) et une distribution/perforation est possible en supprimant tout simplement le doigt de poussée M64.

Sans sortir du cadre de l'invention, il est possible de combiner l'ajustement en profondeur de pénétration avec la permutation. On peut très facilement envisager une troisième position pour le sélecteur M7', dans laquelle le porte-aiguilles N est étendu ou avancé comme dans le premier mode de réalisation. On aurait alors trois positions avec les configurations suivantes :
- Repos : distribution seule ou distribution/perforation,
- Intermédiaire : distribution/perforation ou perforation seule,
- Avancée : distribution/perforation profonde ou perforation profonde seule.

Dans les modes de réalisation qui viennent d'être décrits, les accessoires qui permettent d'alimenter et de commander le moteur ne sont pas représentés. On peut cependant envisager d'utiliser la commande du moteur pour séquencer les phases de distribution et de perforation : la durée et le déroulement des séquences peuvent être contrôlés par un microprocesseur dédié.

Nomenclature des références :
Premier module : M ; M'
Moteur : M2 - Arbre du moteur : M21
Capuchon vacillant : M3 - Tête inclinée: M31
Piston de transmission: M4 ; M4' - Partie inférieure M4a - Partie supérieure :M4b - Conduit d'entrée : M40 - Surface inclinée : M41 - Siège de clapet : M42 - Support de soufflet : M43 - Moyens de transmission : M44 - Extrémité de contact : M45
Pompe à air : P - Chambre de pompe : Cp - Clapet d'entrée : Vi ; Vi' - Clapet de sortie : Vo - Soufflet : B
Virole : M5 - Cylindre : M51 - Gaine d'étanchéité : M52 - Conduit de sortie : M53 - Siège de clapet de sortie : M54 - Support de soufflet : M55 Chambre à air : Ca
Chariot : M6 ; M6' - Panier : M6a - Actionneur de clapet : M6b - Logement : M62 - Téton : M67 - Doigt de poussée : M64
Sélecteur : M7 ; M7' - Chemin de came : M76
Coque externe : M8 - fenêtre latérale M86
Second module (Cartouche) : C
Réservoir de produit fluide : R - Fût de coulissement : R1 - Douille d'étanchéité : R2 - Nervures d'éventation : R3 - Piston pousseur : R4 Corps : C1 - Carénage : C2
Embout d'application : A - Canal de sortie : A1 - Face d'application : AO - Sortie de produit fluide : O
Porte-aiguilles : N - Micro-aiguilles : NO - Face de support : N1 - Moyens de transmission : N2
Ressort de rappel : S

## Revendications

1. Applicateur à micro-aiguilles pour appliquer un produit fluide sur la peau et le faire pénétrer dans la peau, l'applicateur définissant un axe longitudinal X et comprenant :
- une face d'application (AO) pourvue d'au moins une sortie de produit fluide (O),
- plusieurs micro-aiguilles (NO),
- un réservoir de produit fluide (R) relié à la sortie de produit fluide (O),
- un moteur (M2) pour faire vibrer les micro-aiguilles (NO) et acheminer le produit fluide issu du réservoir de produit fluide (R) à la sortie de produit fluide (O),
**caractérisé en ce qu'**il comprend deux modules distincts (M ; M', C) raccordés axialement l'un à l'autre de manière amovible, à savoir :
- un premier module (M ; M') abritant le moteur (M2) et des accessoires pour faire fonctionner le moteur (M2), et
- un second module (C) abritant le réservoir de produit fluide (R), formant la face d'application (AO) et supportant les micro-aiguilles (NO).

2. Applicateur selon la revendication 1, dans lequel le réservoir de produit fluide (R) est à volume variable, l'actionnement du moteur (M2) agissant sur le réservoir de produit fluide (R) sans transmission mécanique de force pour engendrer une diminution de volume du réservoir de produit fluide (R), de sorte qu'une partie de son contenu est refoulée vers la sortie de produit fluide (O).

3. Applicateur selon la revendication 2, dans lequel le premier module (M ; M') comprend une pompe à air (P).

4. Applicateur selon la revendication 3, dans lequel le réservoir de produit fluide (R) comprend une paroi mobile (R4) qui fait aussi partie d'une chambre à air (Ca) alimentée en air pressurisé issu de la pompe à air (P).

5. Applicateur selon la revendication 4, dans lequel la chambre à air (Ca) est formée conjointement par les deux modules (M ; M', C) raccordés de manière étanche.

6. Applicateur selon la revendication 5, dans lequel la pompe à air (P) comprend une chambre de pompe (Cp) dotée d'un clapet d'entrée d'air (Vi ; Vi',) et d'un clapet de sortie d'air (Vo) relié à la chambre à air (Ca), la variation de volume de la chambre de pompe (Cp) étant avantageusement assurée par un soufflet (B).

7. Applicateur selon la revendication 6, dans lequel le clapet d'entrée d'air (Vi ; Vi') est entrainé axialement en va-et-vient par le moteur (M2).

8. Applicateur selon l'une quelconque des revendications précédentes, dans lequel le moteur (M2) comprend un arbre axial (M21) entrainé en rotation, un système de transformation de mouvement rotatif en axial (M3) étant monté sur l'arbre rotatif (M21).

9. Applicateur selon la revendication 6, dans lequel le premier module (M') comprend des moyens de neutralisation (M64) de la pompe à air (P) pour l'empêcher d'alimenter la chambre à air (Ca) en air pressurisé, avantageusement en bloquant son clapet d'entrée d'air (Vi') en position ouverte.

10. Applicateur selon l'une quelconque des revendications précédentes, dans lequel le premier module (M) comprend des moyens d'ajustement (M6, M7) de la profondeur de pénétration des micro-aiguilles (NO), agissant avantageusement sur la position axiale du moteur (M2) dans le premier module (M).

11. Applicateur selon l'une quelconque des revendications précédentes, dans lequel le premier module (M') comprend des moyens d'embrayage/débrayage (M6', M7') pour embrayer/débrayer la transmission des vibrations générées par le moteur (M2) aux micro-aiguilles (NO).

12. Applicateur selon les revendications 9 et 11, dans lequel la pompe à air (P) est neutralisée lorsque les micro-aiguilles (NO) sont embrayées au moteur (M2).

13. Applicateur selon les revendications 9 et 11, dans lequel la pompe à air (P) alimente la chambre à air (Ca) en air pressurisé lorsque les micro-aiguilles (NO) sont débrayées du moteur (M2).

14. Applicateur selon l'une quelconque des revendications précédentes, dans lequel la face d'application (AO) est fixe et les micro-aiguilles (NO) sont montées sur un porte-aiguilles (N) entrainé en va-et-vient par le moteur (M2).

15. Applicateur selon l'une quelconque des revendications précédentes, dans lequel les micro-aiguilles (NO) sont montées sur un porte-aiguilles (N) qui s'étend autour du réservoir de produit fluide (R).

16. Applicateur selon la revendication 6, dans lequel le premier module (M ; M') comprend un piston (M4 ; M4') déplaçable axialement en va-et-vient sous l'action du moteur (M2), ce piston (M4 ; M4') formant un siège (M42) pour le clapet d'entrée d'air (Vi ; Vi'), un support (M43) pour un soufflet (B) et des moyens de transmission (M44) pour entrainer les micro-aiguilles (NO) du second module (C) en vibration.

## Patentansprüche

1. Applikator mit Mikronadeln zum Auftragen eines Fluidprodukts auf die Haut und zum Eindringen des Fluidprodukts in die Haut, wobei der Applikator eine Längsachse X definiert und umfasst:
- eine Applikationsfläche (PO), die mit zumindest einem Fluidproduktauslass (O) versehen ist,
- mehrere Mikronadeln (NO),
- ein Fluidproduktreservoir (R), das mit dem Fluidproduktauslass (O) verbunden ist,
- einen Motor (M2), um die Mikronadeln (NO) in Schwingung zu versetzen und das Fluidprodukt aus dem Fluidproduktreservoir (R) zum Fluidproduktauslass (O) zu leiten,
**dadurch gekennzeichnet, dass** er zwei separate Module (M ; M', C) umfasst, die axial lösbar miteinander verbunden sind, nämlich:
- ein erstes Modul (M; M'), das den Motor (M2) und Zubehörteile zum Betreiben des Motors (M2) aufnimmt, und
- ein zweites Modul (C), das das Fluidproduktreservoir (R) aufnimmt, die Applikationsfläche (AO) bildet und die Mikronadeln (NO) abstützt.

2. Applikator nach Anspruch 1,
wobei das Fluidproduktreservoir (R) ein variables Volumen hat, wobei die Betätigung des Motors (M2) ohne mechanische Kraftübertragung auf das Fluidproduktreservoir (R) einwirkt, um eine Volumenverringerung des Fluidproduktreservoirs (R) zu bewirken, so dass ein Teil seines Inhalts zum Fluidproduktauslass (O) verdrängt wird.

3. Applikator nach Anspruch 2,
wobei das erste Modul (M; M') eine Luftpumpe (P) umfasst.

4. Applikator nach Anspruch 3,
wobei das Fluidproduktreservoir (R) eine bewegliche Wand (R4) umfasst, die auch Teil einer Luftkammer (Ca) ist, die mit Druckluft aus der Luftpumpe versorgt wird.

5. Applikator nach Anspruch 4,
wobei die Luftkammer (Ca) gemeinsam von den beiden abgedichtet angeschlossenen Modulen (M; M', C) gebildet wird.

6. Applikator nach Anspruch 5,
wobei die Luftpumpe (P) eine Pumpenkammer (Cp) mit einem Lufteinlassventil (Vi; Vi') und einem mit der Luftkammer (Ca) verbundenen Luftauslassventil (Vo) umfasst, wobei die Volumenänderung der Pumpenkammer (Cp) vorteilhafterweise über einen Balg (B) sichergestellt wird.

7. Applikator nach Anspruch 6,
wobei das Lufteinlassventil (Vi; Vi') durch den Motor (M2) axial hin- und herbewegt wird.

8. Applikator nach einem der vorhergehenden Ansprüche,
wobei der Motor (M2) eine drehend angetriebene Axialwelle (M21) umfasst, wobei ein System zur Umwandlung von Dreh- in Axialbewegung (M3) an der Drehwelle (M21) angebracht ist.

9. Applikator nach Anspruch 6,
wobei das erste Modul (M') Abstellmittel (M64) zum Abstellen der Luftpumpe (P) umfasst, um sie daran zu hindern, die Luftkammer (Ca) mit Druckluft zu versorgen, vorteilhafterweise durch Sichern ihres Lufteinlassventils (Vi') in der offenen Position.

10. Applikator nach einem der vorhergehenden Ansprüche,
wobei das erste Modul (M) Einstellmittel (M6, M7) zum Einstellen der Eindringtiefe der Mikronadeln (NO) umfasst, die vorteilhaft auf die axiale Stellung des Motors (M2) im ersten Modul (M) einwirken.

11. Applikator nach einem der vorhergehenden Ansprüche,
wobei das erste Modul (M') Zuschalt-/Abschaltmittel (M6', M7') zum Zuschalten/Abschalten der Übertragung der durch den Motor (M2) erzeugten Schwingungen auf die Mikronadeln (NO) umfasst.

12. Applikator nach Anspruch 9 und 11,
wobei die Luftpumpe (P) abgestellt ist, wenn die Mikronadeln (NO) mit dem Motor (M2) gekoppelt sind.

13. Applikator nach Anspruch 9 und 11,
wobei die Luftpumpe (P) die Luftkammer (Ca) mit Druckluft versorgt, wenn die Mikronadeln (NO) vom Motor (M2) abgekoppelt sind.

14. Applikator nach einem der vorhergehenden Ansprüche,
wobei die Applikationsfläche (AO) feststehend ist und die Mikronadeln (NO) an einem Nadelhalter (N) gelagert sind, der vom Motor (M2) hin- und herbewegt wird.

15. Applikator nach einem der vorhergehenden Ansprüche,
wobei die Mikronadeln (NO) an einem Nadelhalter (N) gelagert sind, der sich um das Fluidproduktreservoir (R) herum erstreckt.

16. Applikator nach Anspruch 6,
wobei das erste Modul (M; M') einen Kolben (M4; M4') umfasst, der unter der Wirkung des Motors (M2) axial hin- und herbewegt werden kann, wobei dieser Kolben (M4; M4') einen Sitz (M42) für das Lufteinlassventil (Vi; Vi') bildet, sowie eine Halterung (M43) für einen Balg (B) und Übertragungsmittel (M44), um die Mikronadeln (NO) des zweiten Moduls (C) in Schwingung zu versetzen.

## Claims

1. A microneedle applicator for applying a fluid product on the skin and for making it penetrate into the skin, the applicator defining a longitudinal axis X and comprising:
- an application face (AO) provided with at least one fluid product outlet (0),
- a plurality of microneedles (NO),
- a fluid product reservoir (R) connected to the fluid product outlet (O);
- a motor (M2) for causing the microneedles (NO) to vibrate and to convey the fluid product from the reservoir (R) to the outlet (O);
and being **characterised in that** it comprises two distinct modules (M; M'; C) axially and removably connected to each other, namely:
- a first module (M; M') housing the motor (M2) and accessories for operating the motor (M2), and
- a second module (C) housing the fluid product reservoir (R), forming the application face (AO) and supporting the microneedles (NO).

2. The applicator according to Claim 1, wherein the fluid product reservoir (R) has a variable volume, the actuation of the motor (M2) acting on the fluid product reservoir (R) without any mechanical transmission, to lead to a decrease in volume of the fluid product reservoir (R), such that some of its content is repelled to the fluid product outlet (O).

3. The applicator according to Claim 2, wherein the first module (M; M') comprises an air pump (P).

4. The applicator according to Claim 3, wherein the fluid product reservoir (R) comprises a movable wall (R4) that also forms part of an air chamber (Ca) supplied with pressurised air from the air pump (P).

5. The applicator according to Claim 4, wherein the air chamber (Ca) is formed jointly by the two modules connected in a sealed manner (M; M', C).

6. The applicator according to Claim 5, wherein the air pump (P) comprises a pump chamber (Cp) equipped with an air inlet valve (Vi; Vi',) and an air outlet valve (Vo) connected to the air chamber (Ca), the variation in volume of the pump chamber (Cp) being advantageously provided by a bellows (B).

7. The applicator according to Claim 6, wherein the air inlet valve (Vi; Vi') is driven axially back and forth by the motor (M2).

8. The applicator according to any one of the preceding claims, wherein the motor (M2) comprises an axial shaft (M21) driven in rotation, a rotary to axial motion transformation system (M3) being mounted on the rotary shaft (M21).

9. The applicator according to Claim 6, wherein the first module (M') comprises means (M64) for neutralising the air pump (P) to prevent it from supplying the air chamber (Ca) with pressurised air, advantageously by blocking its air inlet valve (Vi') in the open position.

10. The applicator according to any one of the preceding claims, wherein the first module (M) comprises means (M6, M7) for adjusting the penetration depth of the microneedles (NO), acting advantageously on the axial position of the motor (M2) in the first module (M).

11. The applicator according to any one of the preceding claims, wherein the first module (M') comprises engagement/disengagement means (M6', M7') for engaging/disengaging the transmission of the vibrations generated by the motor (M2) to the microneedles (NO).

12. The applicator according to Claims 9 and 11, wherein the air pump (P) is neutralised when the microneedles (NO) are engaged with the motor (M2).

13. The applicator according to Claims 9 and 11, wherein the air pump (P) supplies the air chamber (Ca) with pressurised air when the microneedles (NO) are disengaged from the motor (M2).

14. The applicator according to any one of the preceding claims, wherein the application face (AO) is attached and the microneedles (NO) are mounted on a needle holder (N) driven back and forth by the motor (M2).

15. The applicator according to any one of the preceding claims, wherein the microneedles (NO) are mounted on a needle holder (N) which extends around the fluid product reservoir (R).

16. The applicator according to Claim 6, wherein the first module (M; M') comprises a piston (M4; M4') which may be moved axially back and forth under the action of the motor (M2), this piston (M4; M4') forming a seat (M42) for the air inlet valve (Vi; Vi'), a support (M43) for a bellows (B), and transmission means (M44) for driving the microneedles (NO) of the second module (C) in vibration.
